# EUROPEAN PATENT SPECIFICATION

(11) **EP 4 251 065 B1**
(45) Date of publication and mention of the grant of the patent: **07.10.2026**
(21) Application number: 21844088.1
(22) Date of filing: 29.11.2021
(51) Int. Cl.: A61B 5/07, A61B 5/20

(54) **IMPLANTABLE PASSIVE MEAN PRESSURE SENSOR**
IMPLANTIERBARER PASSIVER MITTELDRUCKSENSOR
CAPTEUR PASSIF IMPLANTABLE DE PRESSION MOYENNE

(30) Priority: 30.11.2020 US 202063119337 P
(43) Date of publication of application: 04.10.2023
(73) Proprietor: Boston Scientific Scimed Inc., Maple Grove, Minnesota 55311 (US)
(72) Inventor: STAHMANN, Jeffrey E., Ramsey, Minnesota 55303 (US)
(74) Representative: Peterreins Schley
(86) International application number: PCT/US2021/060990
(87) International publication number: WO 2022/115690

(56) References cited:
- WO-A1-2019/136218
- US-A- 4 899 751
- US-A- 5 693 886
- US-A1- 2004 147 969
- US-A1- 2007 255 112
- US-A1- 2009 312 650
- US-A1- 2010 185 103
- US-A1- 2013 165 801
- US-A1- 2019 336 135
- US-B1- 8 142 363

## Description

### Cross Reference to Related Applications

This application claims the benefit of U.S. Provisional Patent Application Serial No. 63/119,337 filed on November 30, 2020.

### Technical Field

The present disclosure pertains to pressure sensors. More particularly, the present disclosure pertains to implantable pressure sensors that can provide a mean pressure indication.

### Background

A variety of pressure sensors have been developed for measuring a variety of different pressures, including blood pressure. Some pressure sensors may be implanted in the body in order to provide blood pressure values. Because they are implanted, there can be issues with being able to provide power to the pressure sensor over time. Moreover, while pressure sensors can provide an instantaneous indication of pressure, they typically are not configured to provide an indication of mean pressure over time. In US 2009/312650 A1 methods for activating implantable medical devices within a patient's body are disclosed. An illustrative method includes activating an implantable medical device from a low-power state to an awake state in response to a scheduled time event, sensing one or more pressure measurements within the body, computing an average pressure measurement based on the sensed pressure measurements, storing the average pressure measurement within a memory of the implantable medical device, and then returning the device to the low-power state. A triggering event such as the detection of patient activity or motion can also be used to activate the implantable medical device between the low-power state and an active state.

In US 2007/255112 detection of volume depletion, particularly after an incidence of volume overload is disclosed. Various methods, systems, and devices are disclosed that sense and analyze a physiological parameter related to a patient's fluid level in order to warn patients of potentially dangerous volume depletion conditions while minimizing false notifications.

US 4 899 751 A discloses a system for therapeutic stimulation of a patient's heart that includes means for varying the escape interval or rate of a means for producing pacing pulses. Pressure sensing means sense pressure at a site in the circulatory system of a patient. Signal producing means respond to output from the sensing means to develop a variable first signal representative of mean pressure at the site over a period of predetermined duration. Signal producing means respond to output from the sensing means for developing a second signal representing mean pressure at the site over a period of given duration less than the period of predetermined duration. Control signal producing means responsive to the first signal and to the second signal develop a control signal upon the first signal and the second signal differing by at least a predetermined amount. Control means, responsive to the control signal, control the means for varying the escape interval or rate of the means for producing pacing pulses. The escape interval or rate may be additionally controlled by a second control signal related to the activity level of the patient either conjointly or contemporaneously. A method of therapeutic stimulation of a patient's heart is also disclosed. The method can be viewed as an analogue of the system.

US 2004/147969 A1 discloses an apparatus for treating cardiovascular disease in a medical patient that includes one or more sensors, an implantable housing, at least one implantable lead, a signal processor, and a signaling device. The sensor is operable to generate a sensor signal indicative of fluid pressure within the left atrium of the heart. The implantable housing includes a cardiac rhythm management apparatus, such as a pacemaker or a defibrillator. The cardiac rhythm management apparatus includes an electrode, which is operable to deliver an electrical stimulus to a location in the heart. The electrical stimulus is delivered based at least in part on the sensor signal. The lead is coupled to the implantable housing, and to the electrode. The signal processor is operable to generate a processor output indicative of a treatment, and is based at least in part on the sensor signal. The signaling device is operable to generate at least two distinct treatment signals distinguishable from one another by the patient. Each signal is indicative of a different therapeutic treatment; and is based at least in part on the processor output

US 2013/0165801 A1 discloses a passive pressure sensor used with an implantable lead to measure pressure within a patient's heart. In some embodiments, the passive pressure sensor is incorporated into an implantable lead. In other embodiments, the passive pressure sensor is incorporated into a device that is slid onto an implantable lead.

WO 2019/136218 A1 discloses a system for treating and/or monitoring a medical condition of a patient comprising an implant configured to be inserted into a body space of the patient, such as the left atrial appendage. The implant comprises a frame or ring configured to engage tissue of the patient proximate the body space. Methods of treating and/or monitoring a medical condition of a patient are also provided.

US 5,693,886 discloses a pressure sensor, having a simple structure for accurately measuring any one of pressure and weight The pressure sensor includes a pair of electrode layers and a dielectric layer. The dielectric layer is composed of a rubber elastic body positioned between the pair of electrode layers to act as a spacer for the pair of electrode layers. The dielectric layer has a tan δ, at 1 to 30 Hz at a temperature of 10°C. to 30°C., of 0.03 or less. The dielectric layer also has a rubber hardness of 20 to 80 degrees in terms of scale A according to JIS-K-6301 at 10°C. to 30°C. Further, the dielectric layer has an impact resilience of 75% or more measured according to JIS-K-6301 at 10°C. to 30°C. and a compression set of 3% or less measured according to JIS-K-6301 at 10°C. to 30°C. The dielectric layer is formed of any of natural rubber, polybutadiene rubber, polyisoprene rubber, polyurethane rubber and silicone rubber.

There is an ongoing need for a pressure sensor that can provide an indication of mean pressure over time, particularly without requiring ongoing electrical energy in order to maintain a measurement of mean pressure over time.

### Summary

This disclosure pertains to pressure sensors that can provide an indication of mean pressure over time, even without ongoing electrical energy to maintain the indication of mean pressure over time. As an example, an implantable pressure sensor includes a housing and a sensing component that is housed by the housing and exposed to an external pressure that is exterior to the housing. In response to the external pressure, the sensing component is configured to undergo a physical transformation that is representative of a mean pressure of the external pressure over a period of time of at least one minute. The pressure sensor includes circuitry that is operatively coupled to the sensing component and that is responsive to the physical transformation of the sensing component to produce a mean pressure signal that is representative of the mean pressure of the external pressure over the period of time.

Alternatively or additionally, the circuitry may be configured to repeatedly receive an energy transfer from a remote source, and in response, transmit the mean pressure signal back to the remote source.

Alternatively or additionally, the sensing component may undergo and maintain the physical transformation that is representative of the mean pressure of the external pressure over the period of time without the application of electrical power.

Alternatively or additionally, the physical transformation of the sensing component may have a time constant of at least one hour.

Alternatively or additionally, the physical transformation of the sensing component may have a time constant of at least twelve hours.

Alternatively or additionally, the physical transformation of the sensing component may have a time constant of at least 1 day.

Alternatively or additionally, the housing may be adapted for being disposed within a patient's heart, bladder, brain, spinal column, eye, joint or vascular system.

Alternatively or additionally, the sensing component may include a first plate exposed to the external pressure exterior to the housing and configured to move in response to pressure changes within the external pressure, a second plate spaced apart from the first plate and disposed within the housing, and a viscoelastic material disposed between the first plate and the second plate.

Alternatively or additionally, the viscoelastic material may include a non-conductive material, and the mean pressure signal may be representative of a capacitance between the first plate and the second plate.

Alternatively or additionally, the viscoelastic material may include an electrically conductive material, and the mean pressure signal may be an indication of a resistance between the first plate and the second plate.

Alternatively or additionally, the first plate may be electrically conductive and the second plate may be electrically conductive.

Alternatively or additionally, the sensing component may include a hydraulic sensing component.

Alternatively or additionally, the hydraulic sensing component may include a hydraulic chamber exposed to the external pressure, a first fluid disposed within the hydraulic chamber, a pressure chamber, a second fluid disposed within the pressure chamber, and a barrier disposed between the first fluid and the second fluid. Pressure changes in the hydraulic chamber relative to a pressure within the pressure chamber may cause movement of the barrier.

Alternatively or additionally, the barrier may include a low flow valve with a high permeability material.

As another example, an implantable pressure sensor is configured to provide a signal providing an indication of a mean pressure value over a period of time. The implantable pressure sensor includes a housing configured to be implanted in a patient and a sensing component disposed within the housing yet exposed to an environment exterior to the housing. The sensing component is configured to maintain a mean pressure measurement providing an indication of a mean pressure within the environment exterior to the housing over a period of time without electrical energy.

Alternatively or additionally, the sensing component may include a viscoelastic material having a time constant of at least one hour.

As another example, a left atrial appendage closure device is adapted to fit within a patient's left atrial appendage in order to substantially close off the patient's left atrial appendage. The left atrial appendage closure device includes an expandable framework. A sensing component is exposed to a left atrium pressure within the left atrium of the patient's heart once the left atrial appendage closure device is implanted, wherein in response to the left atrium pressure, the sensing component is configured to undergo a physical transformation that is representative of a mean pressure of the left atrium pressure over a period of time of at least 1 minute. Circuitry is operatively coupled to the sensing component and is responsive to the physical transformation of the sensing component to produce a mean pressure signal that is representative of the mean pressure of the left atrium pressure over the period of time.

Alternatively or additionally, the sensing component may include a viscoelastic material with a time constant of at least 10 minutes that, in response to the left atrium pressure, viscoelastically deforms in a manner that represent the mean pressure of the left atrium pressure over the period of time.

Alternatively or additionally, the sensing component may include a hydraulic system including a low flow valve, wherein in response to a positive change in the left atrium pressure, the sensing component moves fluid through the low flow valve under a hydraulic resistance in a first direction subject to a time constant of the sensing component defined at least in part by the low flow valve.

Alternatively or additionally, in response to a negative change in the left atrium pressure, the sensing component may move fluid through the low flow valve in a second direction opposite to the first direction subject to the time constant of the sensing component.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a schematic block diagram of an illustrative pressure sensing system;
FIG. 2 is a schematic diagram of an illustrative pressure sensor;
FIG. 3 is a graphical representation of mean pressure data;
FIG. 4 is a schematic diagram of an illustrative pressure sensor;
FIG. 4A is a schematic diagram of a portion of an illustrative pressure sensor;
FIG. 4B is a schematic diagram of a portion of an illustrative pressure sensor;
FIG. 5 is a perspective view of an illustrative left atrial appendage closure device; and
FIG. 6 is a side view of the illustrative left atrial appendage closure device of FIG. 5.

While the disclosure is amenable to various modifications and alternative forms, specifics thereof have been shown by way of example in the drawings and will be described in detail. It should be understood, however, that the invention is defined by the appended claims.

### Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

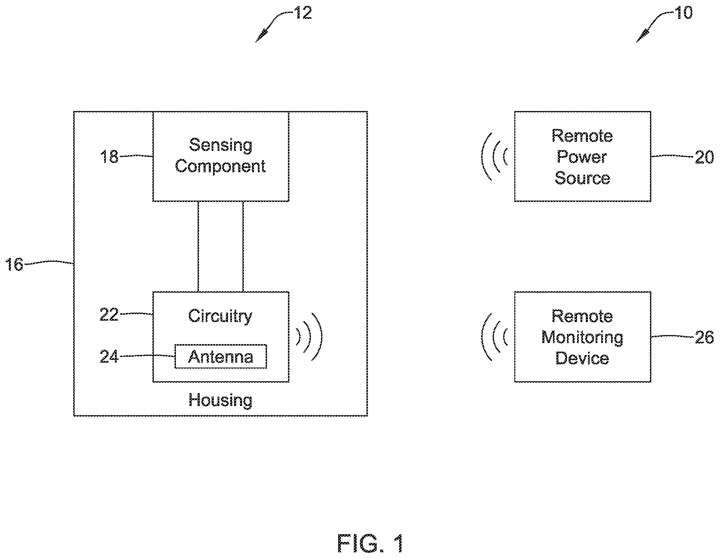

FIG. 1 is a schematic block diagram of an illustrative pressure sensing system 10. The pressure sensing system 10 includes a pressure sensor 12 and a remote energy source 20. The pressure sensor 12 may be configured to be implanted within a patient's body. In some cases, the pressure sensor 12 may be configured to be implanted at a location in which the pressure sensor 12 may be exposed to the patient's blood, such as within the patient's vasculature. For example, the pressure sensor 12 may be configured to be implanted within a chamber of the patient's heart. In other cases, the pressure sensor 12 may be configured to be implanted within a patient's bladder, artery, vein, brain, spinal column, eye, joint or other location, for example.

The illustrative pressure sensor 12 includes a housing 16 that is configured to be implanted at a desired location within the patient. While the pressure sensor 12 is described herein as being deployed as an implantable device, intended for use in the human body, it will be appreciated that the pressure sensor 12 may be applied to other applications as well. When intended for use in the human body, the housing 16 may be formed of any suitable biocompatible material, such as a biocompatible polymeric material or a biocompatible metallic material. In some cases, the housing 16 may be formed of a material that is not biocompatible, but may have a biocompatible film or coating covering an exterior of the housing 16.

The pressure sensor 12 may be considered as being configured to provide an indication of a mean pressure, or an average pressure value over time. The mean pressure may be rolling mean pressure taken over a predetermined time horizon. That is, rather than an instantaneous pressure sensor that immediately reports a currently detected pressure, the pressure sensor 12 creates and maintains a mean pressure measurement that is not overly influenced by transient spikes in pressure. For example, a person's blood pressure may spike while they are climbing stairs, or otherwise exerting themselves. An instantaneous blood pressure measurement, especially when taken only at certain time intervals, may not provide an accurate picture of the person's overall cardiovascular health. In contrast, a mean blood pressure measurement may do a better job of ignoring short term pressure spikes to more accurately represent an overall blood pressure trend.

The illustrative pressure sensor 12 includes a sensing component 18. The sensing component 18 may be configured to create and maintain an indication of mean pressure that represents a mean pressure of the environment outside of the housing 16 over time. To that end, the housing 16 may be configured to allow the sensing component 18 or at least a portion thereof to be exposed to the environment outside of the housing 16. In some cases, the sensing component 18 may include a viscoelastic material. A viscoelastic material is a material for which the relationship between stress and strain depends on time, and that includes properties of both viscous and elastic materials. Viscosity is a measure of a fluid's resistance to flow while elasticity is a measure of a material's ability to return to its original shape after application of a force. Sorbothane^{®}, which is a highly damped viscoelastic polymeric solid that flows like a liquid under load is one example viscoelastic material. Sorbothane^{®} is a thermoset, polyether-based, polyurethane material. Memory foam is another example viscoelastic material. Other example viscoelastic materials include some polymers, semi-crystalline polymers, biopolymers, neoprene, and bitumen materials. These materials can be tailored to have a desired time constant to match the desired application.

Rather than using a viscoelastic material, the sensing component 18 may be implemented as a hydraulic system that can track a mean or rolling average of pressure over time. Other systems may also be used. In any event, the sensing component 18 may be considered as being configured to maintain an indication of mean pressure over time without needing any source of electricity to create and maintain the indication of mean pressure. As a result, the pressure sensor 12 does not need any on-board source of power, such as a battery. Rather, the pressure sensor 12 may be configured to periodically receive power transmitted from a remote power source 20. In order for the pressure sensor 12 to be able to receive and utilize the power transmitted from the remote power source 20, the pressure sensor 12 includes circuitry 22 that is operably coupled to the sensing component 18.

The illustrative circuitry 22 includes an antenna 24 that is configured to receive electrical energy from the remote power source 20. The circuitry 22 is configured to convert the electrical energy into an appropriate form, if appropriate, and to use the energy to solicit a signal from the sensing component 18. The circuitry 22 then transmits the signal that provides an indication of the mean pressure measured over time by the sensing component 18 to a remote monitoring device 26. In some cases, a single antenna 24 is able to both receive electrical energy transmitted by the remote power source 20 and to communicate with the remote monitoring device 26. In some instances, the antenna 24 may instead represent a first antenna that is configured to receive electrical energy transmitted by the remote power source 20 and a second antenna that is configured to transmit a signal to the remote monitoring device 26. While the remote power source 20 and the remote monitoring device 26 are illustrated as being separate devices, it will be appreciated that in some cases the remote power source 20 and the remote monitoring device 26 may be incorporated into a single hand-held apparatus.

It should be noted that the electrical energy provided to the pressure sensor 12 from the remote power source 20 is not used to create or maintain an indication of a mean pressure measurement over time. Rather, the indication of the mean pressure measurement over time is created and maintained over time by the sensing component 18 without need for ongoing electrical energy. In this example, the electrical energy provided by the remote power source 20 is only used to query the sensing component 18 for an indication of the mean pressure measurement over time and to transmit a signal representing the indication of the mean pressure measurement over time to the remote monitoring device 26.

In some cases, the sensing component 18 may be considered as having being configured to undergo a physical transformation in response to being exposed to an external pressure outside of the housing 16. The physical transformation may be considered as being representative of a mean pressure of the external pressure over a period of at least one minute. Brief spikes in pressure may be largely ignored. The physical transformation of the sensing component 18 may be considered as having a time constant of at least one hour. The physical transformation of the sensing component 18 may be considered as having a time constant of at least twelve hours. The physical transformation of the sensing component 18 may be considered as having a time constant of at least one day. The time constant here represents the speed at which the sensing component 18 can respond to a change in input pressure, defined here as the time for the mean pressure reported by the sensing component 18 to vary by a factor of 1- 1/ e (approximately 0.6321) in response to a step change in input pressure. That is, for a time constant of one day, when the input pressure P is halved to P/2 at time "t" and is maintained at P/2 for a day, the sensing component 18 would report a mean pressure of P - (P/2 * (1-1/e)) ≈ 0.68P after one day. This is just an example.

In some cases, a time constant for increased pressure (e.g. a compression time constant) may be configured to be about equal to a time constant for decreased pressure (e.g. expansion time constant) to facilitate the measurement of a mean pressure measurement. In some cases, the time constant for increased pressure and the time constant for decreased pressure may not be equal. For example, there may be a desire to bias the measurement towards higher or lower pressures, or when both time constants are adjusted to account for biological effects (e.g. tissue encapsulation).

In some cases, the signal provided by the circuitry 22, which is representative of the mean pressure measurement provided by the sensing component 18, may be corrected for sensor hysteresis. This correction may occur within the remote monitoring device 26 in order to reduce the computational requirements for the circuitry 22, although in some cases the circuitry 22 may itself correct for sensor hysteresis. In some instances, a second or additional pressure sensor(s) (not illustrated) may also be implanted within a patient in order to provide an instantaneous measurement when desired, in addition to the mean pressure measurement provided by the pressure sensor 12. The second or additional pressure sensor(s) may be part of the pressure sensor 12 and operatively coupled to the circuitry 22. Alternatively, the additional pressure sensor(s) may be separately housed. The additional pressure sensor(s) may be used to calibrate the pressure sensor 12. Alternatively, the pressure sensor 12 may be used to calibrate the instantaneous pressure sensor. It will be appreciated that the pressure sensor 12 (and any additional pressure sensors) may be configured to operate within the range of pressures that are encountered within a vessel or chamber within the human body. For example, the expected absolute pressure range within the left atrium is 450 to 950 millimeters (mm) Hg. The expected gauge pressure within the left atrium is 2 to 90 mm Hg.

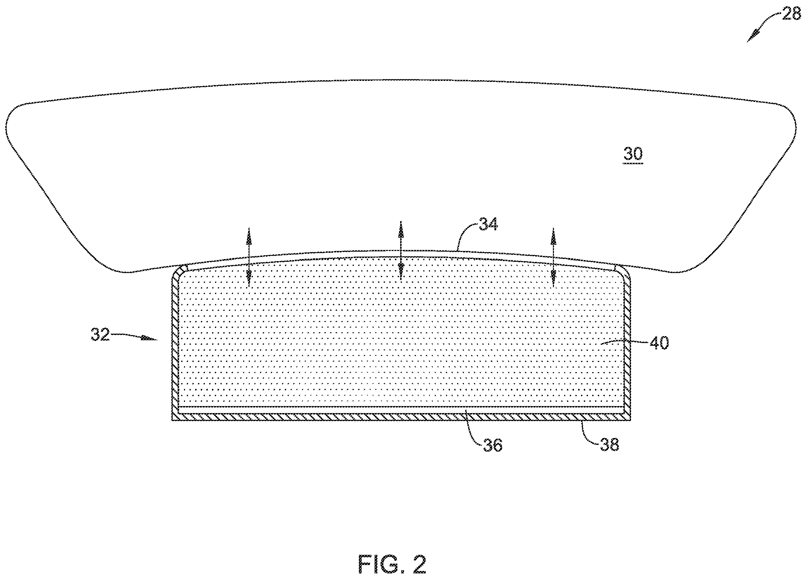

FIG. 2 is a schematic diagram of an illustrative pressure sensor 28 that is exposed to an environment 30 having a variable pressure. The pressure sensor 28 may be considered as an example of the pressure sensor 12. The environment 30 may represent part of the patient's vasculature, including their heart, or the patient's bladder or other cavity. The illustrative pressure sensor 28 includes a chamber 32 that is formed between a first plate 34 and a second plate 36. A housing 38, which may be considered as illustrative of the housing 16 shown in FIG. 1, may provide sides and a bottom to the chamber 32. The first plate 34 is positioned such that changes in pressure within the environment 30 may cause the first plate 34 to flex downward (in the illustrated orientation) when the external pressure within the environment 30 increases, and may cause the first plate 34 to flex upward (in the illustrated orientation) when the external pressure within the environment 30 decreases. In this example, the chamber 32 is filled with a viscoelastic material 40.

The first plate 34 and the second plate 36 may be considered as being electrically conductive. In some cases, when the viscoelastic material 40 is not electrically conductive, the physical transformations that have occurred over time to the viscoelastic material 40 may be indicated by detecting a capacitance between the first plate 34 and the second plate 36. In other cases, when the viscoelastic material 40 is electrically conductive, the physical transformations that have occurred over time to the viscoelastic material 40 may be indicated by detecting a resistance between the first plate 34 and the second plate 36. These are just examples.

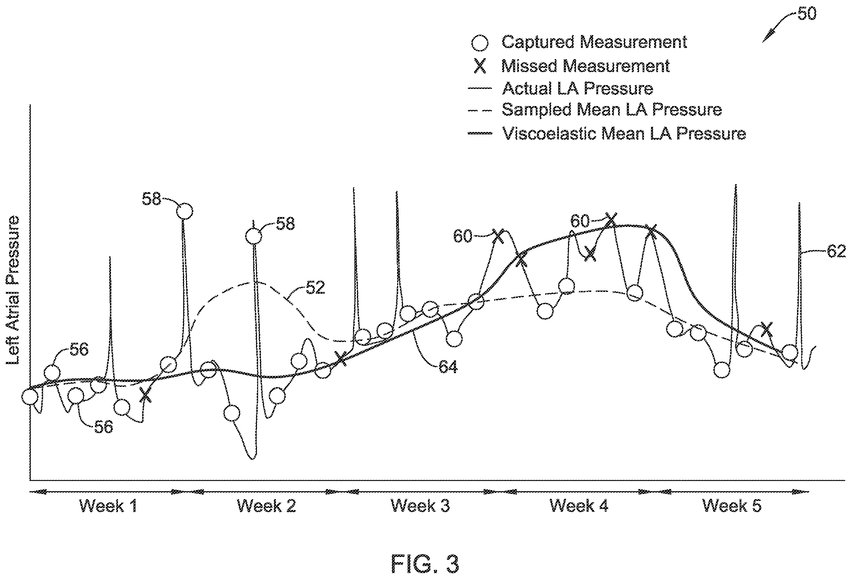

FIG. 3 is a graphical representation 50 of how a viscoelastic material may be used to track a mean pressure measurement over time. This particular example tracks left atrial pressure over a time period of five weeks, with the left atrial pressure indicated on the vertical axis while time is indicated on the horizontal axis. This pressure data is not actual pressure data taken from a patient, but rather has been generated to illustrative how a mean pressure measurement over time is able to ignore transient spikes and missed measurements. While this example is specific to use of a viscoelastic material as part of the sensing component 18, it will be appreciated that the lessons taught by this example are equally applicable to other types of the sensing component 18, such as a hydraulic or other system.

A graphed line 52 shows a mean left atrial pressure as determined by periodic instantaneous measurements 56. In some cases, the graphed line 52 indicates that the mean left atrial pressure as determined by periodic instantaneous measurements 56 can be skewed if the instantaneous measurements are taken at a bad time (e.g. at a short term peak). For example, several periodic instantaneous measurements 58 show left atrial pressure values that are high due to a condition that interferes with the desired mean pressure measurement, such as when an instantaneous measurement was taken while the person was exercising. The graphed line 52 also shows that the mean left atrial pressure as determined by periodic instantaneous measurements may be skewed if instantaneous measurements are missing, such as the missed samples 60. A graphed line 62 provides a representation of the patient's actual left atrial pressure. It can be seen that the graphed line 62 passes through each of the periodic instantaneous measurements 56, the badly timed instantaneous measurements 58 and the missed instantaneous measurements 60. It can be seen that the actual pressure can periodically spike.

A graphed line 64 provides a representation of the mean left atrial pressure as determined using a viscoelastic material. It can be seen that the graphed line 64 overall tracks the actual left atrial pressure as indicated by the graphed line 62, and the graphed line 64 is not influenced by the poorly timed instantaneous measurements 58 or by the missed instantaneous measurements 60. This illustrates how the mean pressure measurement may be used as a reliable indication of blood pressure performance without requiring frequent instantaneous measurements. This also illustrates how a viscoelastic material may be used to provide an indication of mean pressure over time in a manner that does not require electrical energy to either create or maintain the indication of mean pressure over time.

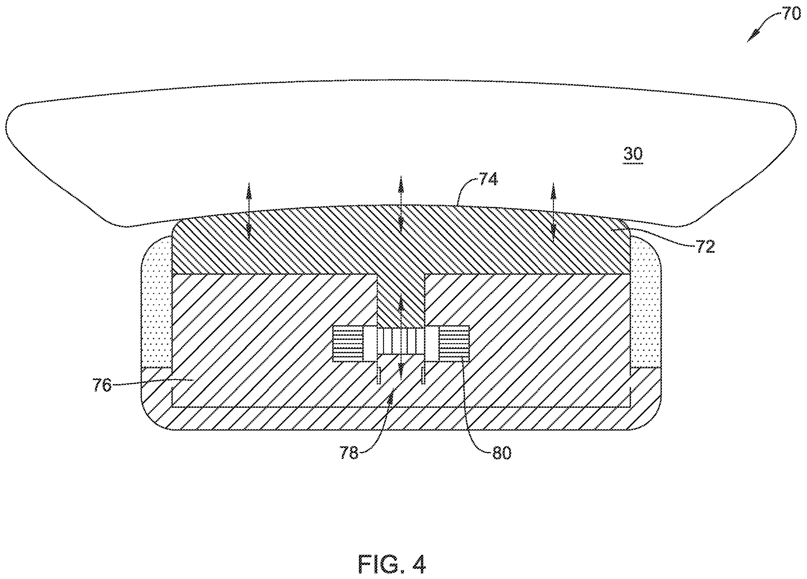

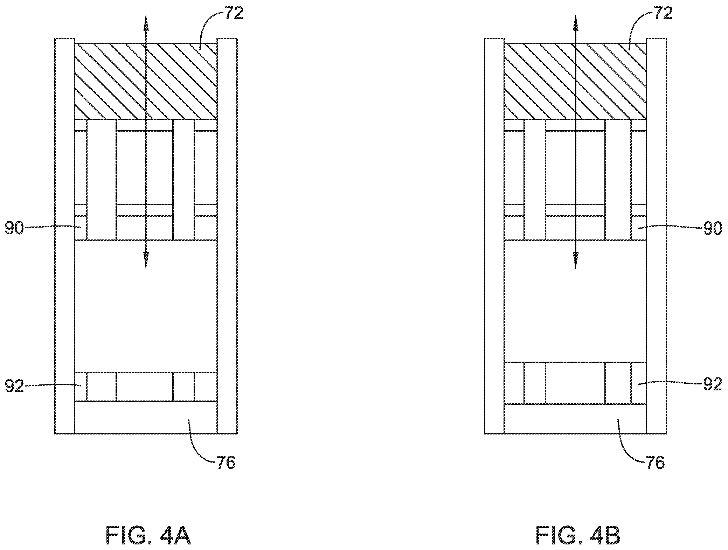

FIG. 4 is a schematic diagram of an illustrative pressure sensor 70 that is exposed to an environment 30 having a variable pressure. The pressure sensor 70 may be considered as an example of the pressure sensor 12, and may operate hydraulically. The pressure sensor 70 includes a hydraulic chamber 72 that is exposed to the external pressure within the environment 30. The hydraulic chamber 72 includes a chamber wall 74 that is configured to bend and flex in response to relative pressure differences between the environment 30 and the hydraulic chamber 72. The hydraulic chamber 72 includes a first fluid. The pressure sensor 70 includes a pressure chamber 76 that includes a second fluid. The second fluid may be the same as the first fluid. In some cases, the first fluid may be a liquid. In some cases, the second fluid may be a liquid. A barrier 78 is fluidly coupled between the hydraulic chamber 72 and the pressure chamber 76. The hydraulic chamber 72 may be considered as acting as a movement multiplier, such that relatively small pressure differences between the environment 30 and the hydraulic chamber 72 result in detectable movement of the barrier 78.

In the example shown, the barrier 78 includes a low flow valve with a high permeability material. As the external pressure within the environment 30 changes, the barrier 78 fluidly disposed between the hydraulic chamber 72 and the pressure chamber 76 moves in response thereto. Also, a maintained external pressure causes some of the fluid to slowly flow through the low flow valve, moving between the hydraulic chamber 72 and the pressure chamber 76. The rate of flow through the low flow valve of the barrier 78 may be chosen to set the time constant of the pressure sensor 70. This physical transformation of the barrier 78 and movement of the fluid between the hydraulic chamber 72 and the pressure chamber 76 is representative of the mean external pressure. To read the mean pressure value, a coil 80 may be coupled with the barrier 78. As the barrier 78 moves in response to pressure changes, the inductance of the coil 80 will change. Accordingly, measuring the inductance of the coil 80 may provide an indication of the mean pressure measurement when the hydraulic chamber 72, the pressure chamber 76 and the fluids therein are non-conductive.

FIG. 4A illustrates a version of the pressure sensor 70 in which capacitance is used to provide an indication of mean pressure. In FIG. 4A, a first plate 90 and a second plate 92 are conductive and function as the barrier 78 (FIG. 4) with a low flow valve. In some cases, the first plate 90 moves in response to changes in mean pressure while the second plate 92 does not. As the distance between the first plate 90 and the second plate 92 varies in accordance with pressure changes, the capacitance between the first plate 90 and the second plate 92 may be used as an indication of the mean pressure measurement. In FIG. 4A, the first and second fluids are electrically non-conductive or substantially electrically non-conductive. In this, substantially electrically non-conductive means that the fluids are not sufficiently electrically conductive to interfere with a capacitance measurement. In FIG. 4B, electrically conductive liquids are used, and changes in resistance between the first plate 90 and the second plate 92 may be used as an indication of the mean pressure measurement.

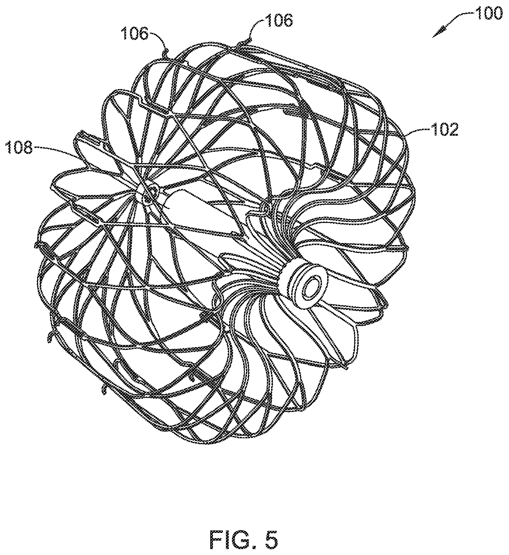

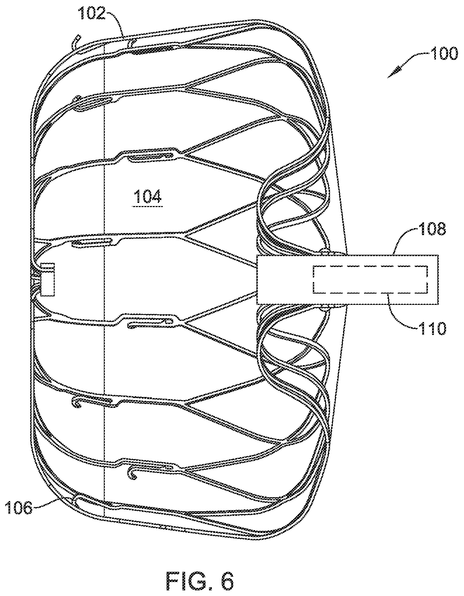

FIG. 5 is a perspective view of an illustrative left atrial appendage closure device 100 while FIG. 6 is a side view thereof showing the left atrial appendage closure device 100 including an occlusive element 104. The left atrial appendage closure device 100 includes an expandable framework 102 that is configured to convert between a collapsed configuration (for delivery) and an expanded configuration, as shown. In some instances, the expandable framework may be compliant and may substantially conform to and/or be in sealing engagement with the shape and/or geometry of a lateral wall and/or an ostium of a left atrial appendage in the expanded configuration. The left atrial appendage closure device 100 may expand to a size, extent, or shape less than or different from a maximum unconstrained extent, as determined by the surrounding tissue and/or lateral wall of the left atrial appendage. Reducing a thickness of various elements of the expandable framework 102 may increase the flexibility and compliance of the expandable framework 102 and/or the left atrial appendage closure device 100, thereby permitting the expandable framework 102 and/or the left atrial appendage closure device 100 to conform to the tissue around it, rather than forcing the tissue to conform to the expandable framework 102 and/or the left atrial appendage closure device 100.

In some cases, as shown in FIG. 6, the left atrial appendage closure device 100 may optionally include an occlusive element 104 (e.g., a mesh, a fabric, a membrane, and/or other surface treatment) disposed on, disposed over, disposed about, or covering at least a portion of the expandable framework 102. In some embodiments, the occlusive element 104 may be disposed on, disposed over, disposed about or cover at least a portion of an outer (or outwardly facing) surface of the expandable framework 102.

In some embodiments, the expandable framework 102 may include a plurality of anchor members 106 disposed about a periphery of the expandable framework 102 in the expanded configuration. The plurality of anchor members 106 may extend radially outward from the expandable framework 102. In some embodiments, at least some of the plurality of anchor members 106 may each have and/or include a body portion, a tip portion, and a barb projecting circumferentially therefrom. In some embodiments, some and/or each of the plurality of anchor members 106 have at least one barb projecting circumferentially therefrom. The plurality of anchor members 106 may provide an anchoring mechanism to aid in retaining the left atrial appendage closure device 100 at a target site within a patient's anatomy (i.e., the left atrial appendage, for example) in the expanded configuration. However, the barb(s) may be configured, positioned, and/or arranged such that engagement of the barb(s) with surrounding tissue at the target site is minimized. For example, the barb(s) may not puncture, pierce, and/or extend into the surrounding tissue in the expanded configuration. Additionally, in some embodiments, the plurality of anchor members 106 may provide an attachment mechanism for securing the occlusive element 104 to the expandable framework 102.

The left atrial appendage closure device 100 may include a central post 108 from which at least some components of the expandable framework 102 may extend. In some instances, as best seen in Figure 6, the central post 108 may include a pressure sensor 110 disposed within the central post 108. In this, the central post 108 may function at least in part as a housing for the pressure sensor 110. While shown schematically, the pressure sensor 110 may represent the pressure sensor 12 shown in FIG. 1. The pressure sensor 110 may represent the pressure sensor 28 shown in FIG. 2. The pressure sensor 110 may represent the pressure sensor 70 shown in FIG. 4. It will be appreciated that by placing the pressure sensor 110 within the central post 108, or securing the pressure sensor 110 to any other portion of the left atrial appendage closure device 100, it is possible to obtain a mean left atrial pressure measurement over time of the left atrium. In some cases, the pressure sensor 110 may extend a substantial portion of the length of the central post 108, but this is not required.

Because endothelization makes removal of the left atrial appendage closure device 100 difficult, having a pressure sensor such as the pressure sensor 110 that can provide an indication of mean left atrial blood pressure over time, without requiring any onboard power supply, means that there are no concerns with battery life, for example. A doctor or other medical professional can simply use the remote power source 20 (FIG. 1) to periodically provide sufficient power for the circuitry within the power sensor 110 to ascertain a mean left atrial blood pressure measurement as indicated by whatever sensing component is present within the pressure sensor 110. This mean left atrial blood pressure measurement may be communicated to the remote monitoring device 26 and used for by a physician or the like in monitoring, diagnosing and/or treating the patient.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. An implantable pressure sensor (12; 28; 70; 110) comprising:
a housing (16);
a sensing component (18) housed by the housing (16) and exposed to an external pressure exterior to the housing (16), wherein in response to the external pressure, the sensing component (18) is configured to undergo a physical transformation that is representative of a mean pressure of the external pressure over a period of time of at least one minute; and
circuitry (22) operatively coupled to the sensing component (18) and responsive to the physical transformation of the sensing component (18) to produce a mean pressure signal that is representative of the mean pressure of the external pressure over the period of time.

2. The implantable pressure sensor (12; 28; 70; 110) of claim 1, wherein the circuitry (22) is configured to repeatedly receive an energy transfer from a remote source (20), and in response, transmit the mean pressure signal back to the remote source (20).

3. The implantable pressure sensor (12; 28; 70; 110) of any one of claims 1 or 2, wherein the sensing component (18) undergoes and maintains the physical transformation that is representative of the mean pressure of the external pressure over the period of time without the application of electrical power.

4. The implantable pressure sensor (12; 28; 70; 110) of any one of claims 1 to 3, wherein the sensing component (18) comprises:
a first plate (34; 90) exposed to the external pressure exterior to the housing, the first plate (34; 90) configured to move in response to pressure changes within the external pressure;
a second plate (36; 92) spaced apart from the first plate (34; 90) and disposed within the housing (16); and
a viscoelastic material (40) disposed between the first plate (24) and the second plate (36).

5. The implantable pressure sensor (12; 28; 70; 110) of claim 4, wherein the viscoelastic material (40) comprises an electrically non-conductive material, and the mean pressure signal is representative of a capacitance between the first plate (34; 90) and the second plate (36; 92).

6. The implantable pressure sensor (12; 28; 70; 110) of claim 4, wherein the viscoelastic material (40) comprises an electrically conductive material, and the mean pressure signal comprises an indication of a resistance between the first plate (34; 90) and the second plate (36; 92).

7. The implantable pressure sensor (12; 28; 70; 110) of any one of claims 1 to 3, wherein the sensing component (18) comprises a hydraulic sensing component.

8. The implantable pressure sensor (12; 28; 70; 110) of claim 7, wherein the hydraulic sensing component comprises:
a hydraulic chamber (72) exposed to the external pressure;
a first fluid disposed within the hydraulic chamber (72);
a pressure chamber (76);
a second fluid disposed within the pressure chamber (76);
a barrier (78) disposed between the first fluid and the second fluid;
wherein pressure changes in the hydraulic chamber (72) relative to a pressure within the pressure chamber (76) causes movement of the barrier (78).

9. The implantable pressure sensor (12; 28; 70; 110) of claim 8, wherein the barrier (78) comprises a low flow valve with a high permeability material.

10. The implantable pressure sensor (12; 28; 70; 110) of any one of claims 1-9, wherein the sensing component (18) is configured to maintain the physical transformation representing the mean pressure of the external pressure over the period of time wherein the sensing component (18) maintains the mean pressure measurement without electrical energy.

11. The implantable pressure sensor (12; 28; 70; 110) of any one of claims 1-6 or 10, wherein the sensing component (18) comprises a/the viscoelastic material, wherein the viscoelastic material has a time constant of at least one hour.

12. A left atrial appendage closure device (100) adapted to fit within a patient's left atrial appendage in order to substantially close off the patient's left atrial appendage, the left atrial appendage closure device (100) comprising:
expandable framework (102); and
the implantable pressure sensor (12; 28; 70; 110) of any one of claims 1-11;
wherein the sensing component (18) is exposed to a left atrium pressure within the left atrium of the patient's heart once the left atrial appendage closure device is implanted, wherein in response to the left atrium pressure, the sensing component (18) is configured to undergo the physical transformation that is representative of a mean pressure of the left atrium pressure over the period of time of at least 1 minute; and
wherein the circuitry (22) is operatively coupled to the sensing component (18) and responsive to the physical transformation of the sensing component (18) to produce a mean pressure signal that is representative of the mean pressure of the left atrium pressure over the period of time.

13. The left atrial appendage closure device (100) of claim 12, wherein the sensing component (18) comprises a viscoelastic material with a time constant of at least 10 minutes that, in response to the left atrium pressure, viscoelastically deforms in a manner that represents the mean pressure of the left atrium pressure over the period of time.

14. The left atrial appendage closure device (100) of claim 12, wherein the sensing component (18) comprises a hydraulic system including a low flow valve, wherein in response to a positive change in the left atrium pressure, the sensing component (18) moves fluid through the low flow valve under a hydraulic resistance in a first direction subject to a time constant of the sensing component defined at least in part by the low flow valve.

15. The left atrial appendage closure device (100) of claim 12, wherein in response to a negative change in the left atrium pressure, the sensing component (18) moves fluid through the low flow valve in a second direction opposite to the first direction subject to the time constant of the sensing component (18).

## Patentansprüche

1. Implantierbarer Drucksensor (12; 28; 70; 110) umfassend:
ein Gehäuse (16);
eine Erfassungskomponente (18), die in dem Gehäuse (16) angeordnet und gegenüber einem externen Druck außerhalb des Gehäuses (16) exponiert ist, wobei die Erfassungskomponente (18) dazu ausgelegt ist, als Reaktion auf den externen Druck eine physische Umwandlung zu durchlaufen, die einen mittleren Druck des externen Drucks über einen Zeitraum von wenigstens einer Minute darstellt; und
eine Schaltung (22), die an die Erfassungskomponente (18) wirkgekoppelt ist und auf die physische Umwandlung der Erfassungskomponente (18) anspricht, um ein mittleres Drucksignal zu erzeugen, das den mittleren Druck des externen Drucks über den Zeitraum darstellt.

2. Implantierbarer Drucksensor (12; 28; 70; 110) nach Anspruch 1, wobei die Schaltung (22) dazu ausgelegt ist, wiederholt eine Energieübertragung von einer entfernten Quelle (20) zu empfangen und als Reaktion das mittlere Drucksignal zurück zu der entfernten Quelle (20) zu übertragen.

3. Implantierbarer Drucksensor (12; 28; 70; 110) nach einem der Ansprüche 1 oder 2, wobei die Erfassungskomponente (18) die physische Umwandlung durchläuft und aufrechthält, die den mittleren Druck des externen Drucks über den Zeitraum darstellt, ohne dass elektrische Leistung angelegt wird.

4. Implantierbarer Drucksensor (12; 28; 70; 110) nach einem der Ansprüche 1 bis 3, wobei die Erfassungskomponente (18) umfasst:
eine erste Platte (34; 90), die gegenüber dem externen Druck außerhalb des Gehäuses exponiert ist, wobei die erste Platte (34; 90) dazu ausgelegt ist, sich als Reaktion auf Druckänderungen des externen Drucks zu bewegen;
eine zweite Platte (36; 92), die von der ersten Platte (34; 90) beabstandet und in dem Gehäuse (16) angeordnet ist; und
ein viskoelastisches Material (40), das zwischen der ersten Platte (24) und der zweiten Platte (36) angeordnet ist.

5. Implantierbarer Drucksensor (12; 28; 70; 110) nach Anspruch 4, wobei das viskoelastische Material (40) ein elektrisch nicht leitfähiges Material umfasst und das mittlere Drucksignal eine Kapazität zwischen der ersten Platte (34; 90) und der zweiten Platte (36; 92) darstellt.

6. Implantierbarer Drucksensor (12; 28; 70; 110) nach Anspruch 4, wobei das viskoelastische Material (40) ein elektrisch leitfähiges Material umfasst und das mittlere Drucksignal eine Anzeige eines Widerstands zwischen der ersten Platte (34; 90) und der zweiten Platte (36; 92) umfasst.

7. Implantierbarer Drucksensor (12; 28; 70; 110) nach einem der Ansprüche 1 bis 3, wobei die Erfassungskomponente (18) eine hydraulische Erfassungskomponente umfasst.

8. Implantierbarer Drucksensor (12; 28; 70; 110) nach Anspruch 7, wobei die hydraulische Erfassungskomponente umfasst:
eine Hydraulikkammer (72), die gegenüber dem externen Druck exponiert ist;
ein erstes Fluid, das in der Hydraulikkammer (72) angeordnet ist;
eine Druckkammer (76);
ein zweites Fluid, das in der Druckkammer (76) angeordnet ist;
eine Barriere (78), die zwischen dem ersten Fluid und dem zweiten Fluid angeordnet ist;
wobei Druckänderungen in der Hydraulikkammer (72) relativ zu einem Druck in der Druckkammer (76) Bewegung der Barriere (78) bewirken.

9. Implantierbarer Drucksensor (12; 28; 70; 110) nach Anspruch 8, wobei die Barriere (78) ein Niedrigstromventil mit einem Material mit hoher Permeabilität umfasst.

10. Implantierbarer Drucksensor (12; 28; 70; 110) nach einem der Ansprüche 1-9, wobei die Erfassungskomponente (18) dazu ausgelegt ist, die physische Umwandlung, die den mittleren Druck des externen Drucks darstellt, über den Zeitraum aufrecht zu halten, wobei die Erfassungskomponente (18) die mittlere Druckmessung ohne elektrische Energie aufrechthält.

11. Implantierbarer Drucksensor (12; 28; 70; 110) nach einem der Ansprüche 1-6 oder 10, wobei die Erfassungskomponente (18) ein/das viskoelastische Material umfasst, wobei das viskoelastische Material eine Zeitkonstante von wenigstens einer Stunde aufweist.

12. Linkes-Vorhofohr-Verschlussvorrichtung (100), dazu ausgelegt, in das linke Vorhofohr eines Patienten zu passen, um das linke Vorhofohr des Patienten im Wesentlichen abzuschließen, wobei die Linkes-Vorhofohr-Verschlussvorrichtung (100) umfasst:
einen erweiterbaren Rahmen (102); und
den implantierbaren Drucksensor (12; 28; 70; 110) nach einem der Ansprüche 1-11;
wobei die Erfassungskomponente (18) gegenüber einem linken Vorhofdruck in dem linken Vorhof des Herzens des Patienten exponiertist, sobald die Linkes-Vorhofohr-Verschlussvorrichtung implantiert ist, wobei die Erfassungskomponente (18) dazu ausgelegt ist, als Reaktion auf den linken Vorhofdruck die physische Umwandlung zu durchlaufen, die einen mittleren Druck des linken Vorhofdrucks über den Zeitraum von wenigstens 1 Minute darstellt; und
wobei die Schaltung (22) an die Erfassungskomponente (18) wirkgekoppelt ist und auf die physische Umwandlung der Erfassungskomponente (18) anspricht, um ein mittleres Drucksignal zu erzeugen, das den mittleren Druck des linken Vorhofdrucks über den Zeitraum darstellt.

13. Linkes-Vorhofohr-Verschlussvorrichtung (100) nach Anspruch 12, wobei die Erfassungskomponente (18) ein viskoelastisches Material mit einer Zeitkonstante von wenigstens 10 Minuten umfasst, das sich als Reaktion auf den linken Vorhofdruck viskoelastisch auf eine Weise verformt, die den mittleren Druck des linken Vorhofdrucks über den Zeitraum darstellt.

14. Linkes-Vorhofohr-Verschlussvorrichtung (100) nach Anspruch 12, wobei die Erfassungskomponente (18) ein Hydrauliksystem umfasst, das ein Niedrigstromventil umfasst, wobei die Erfassungskomponente (18) als Reaktion auf eine positive Änderung des linken Vorhofdrucks Fluid durch das Niedrigstromventil unter einem hydraulischen Widerstand in eine erste Richtung bewegt, abhängig von einer Zeitkonstante der Erfassungskomponente, die wenigstens zum Teil durch das Niedrigstromventil definiert ist.

15. Linkes-Vorhofohr-Verschlussvorrichtung (100) nach Anspruch 12, wobei die Erfassungskomponente (18) als Reaktion auf eine negative Änderung des linken Vorhofdrucks Fluid durch das Niedrigstromventil in eine zweite Richtung entgegengesetzt zu der ersten Richtung bewegt, abhängig von der Zeitkonstante der Erfassungskomponente (18).

## Revendications

1. Capteur de pression implantable (12 ; 28 ; 70 ; 110) comprenant :
un boîtier (16) ;
un composant de détection (18) logé dans le boîtier (16) et exposé à une pression externe extérieure au boîtier (16), le composant de détection (18) étant configuré pour subir une transformation physique représentative d'une pression moyenne de la pression externe sur une période d'au moins une minute ; et
des circuits (22) fonctionnellement couplés au composant de détection (18) et sensibles à la transformation physique du composant de détection (18) pour produire un signal de pression moyenne représentatif de la pression moyenne de la pression externe sur la période.

2. Capteur de pression implantable (12 ; 28 ; 70 ; 110) selon la revendication 1, dans lequel les circuits (22) sont configurés pour recevoir de manière répétée un transfert d'énergie à partir d'une source distante (20), et en réponse, transmettre en retour le signal de pression moyenne à la source distante (20).

3. Capteur de pression implantable (12 ; 28 ; 70 ; 110) selon l'une quelconque des revendications 1 ou 2, dans lequel le composant de détection (18) subit et conserve la transformation physique qui est représentative de la pression moyenne de la pression externe sur la période sans l'application d'énergie électrique.

4. Capteur de pression implantable (12 ; 28 ; 70 ; 110) selon l'une quelconque des revendications 1 à 3, dans lequel le composant de détection (18) comprend :
une première plaque (34 ; 90) exposée à la pression externe extérieure au boîtier, la première plaque (34 ; 90) étant configurée pour se déplacer en réponse à des variations de pression à l'intérieur de la pression externe ;
une seconde plaque (36 ; 92) espacée de la première plaque (34 ; 90) et disposée à l'intérieur du boîtier (16) ; et
un matériau viscoélastique (40) disposé entre la première plaque (24) et la seconde plaque (36).

5. Capteur de pression implantable (12 ; 28 ; 70 ; 110) selon la revendication 4, dans lequel le matériau viscoélastique (40) comprend un matériau non électroconducteur, et le signal de pression moyenne est représentatif d'une capacité entre la première plaque (34 ; 90) et la seconde plaque (36 ; 92).

6. Capteur de pression implantable (12 ; 28 ; 70 ; 110) selon la revendication 4, dans lequel le matériau viscoélastique (40) comprend un matériau électroconducteur, et le signal de pression moyenne est comprend une indication d'une résistance entre la première plaque (34 ; 90) et la seconde plaque (36 ; 92).

7. Capteur de pression implantable (12 ; 28 ; 70 ; 110) selon l'une quelconque des revendications 1 à 3, dans lequel le composant de détection (18) comprend un composant de détection hydraulique.

8. Capteur de pression implantable (12 ; 28 ; 70 ; 110) selon la revendication 7, dans lequel le composant de détection hydraulique comprend :
une chambre hydraulique (72) exposée à la pression externe ;
un premier fluide disposé à l'intérieur de la chambre hydraulique (72) ;
une chambre de pression (76) ;
un second fluide disposé à l'intérieur de la chambre de pression (76) ;
une barrière (78) disposée entre le premier fluide et le second fluide ;
dans lequel des variations de pression dans la chambre hydraulique (72) par rapport à une pression à l'intérieur de la chambre de pression (76) provoquent un mouvement de la barrière (78).

9. Capteur de pression implantable (12 ; 28 ; 70 ; 110) selon la revendication 8, dans lequel la barrière (78) comprend une valve à faible débit avec un matériau à perméabilité élevée.

10. Capteur de pression implantable (12 ; 28 ; 70 ; 110) selon l'une quelconque des revendications 1 à 9, dans lequel le composant de détection (18) est configuré pour conserver la transformation physique représentant la pression moyenne de la pression externe sur la période pendant laquelle le composant de détection (18) conserve la mesure de pression moyenne sans énergie électrique.

11. Capteur de pression implantable (12 ; 28 ; 70 ; 110) selon l'une quelconque des revendications 1 à 6 ou 10, dans lequel le composant de détection (18) comprend un/le matériau viscoélastique, dans lequel le matériau viscoélastique a une constante de temps d'au moins une heure.

12. Dispositif de fermeture d'appendice atrial gauche (100) conçu pour s'adapter à l'intérieur de l'appendice atrial gauche d'un patient dans le but de fermer sensiblement l'appendice atrial gauche du patient, le dispositif de fermeture d'appendice atrial gauche (100) comprenant :
une structure extensible (102) ; et
le capteur de pression implantable (12 ; 28 ; 70 ; 110) selon l'une quelconque des revendications 1 à 11 ;
dans lequel le composant de détection (18) est exposé à une pression de l'atrium gauche à l'intérieur de l'atrium gauche du cœur du patient une fois que le dispositif de fermeture de l'appendice atrial gauche est implanté, dans lequel le composant de détection (18) est configuré pour subir la transformation physique représentative d'une pression moyenne de la pression atriale gauche sur la période d'au moins 1 minute ; et
dans lequel les circuits (22) sont fonctionnellement couplés au composant de détection (18) et sensibles à la transformation physique du composant de détection (18) pour produire un signal de pression moyenne représentatif de la pression moyenne de la pression atriale gauche sur la période.

13. Dispositif de fermeture d'appendice atrial gauche (100) selon la revendication 12, dans lequel le composant de détection (18) comprend un matériau viscoélastique avec une constante de temps d'au moins 10 minutes qui, en réponse à la pression de l'atrium gauche, se déforme viscoélastiquement d'une manière qui représente la pression moyenne de la pression atriale gauche sur la période.

14. Dispositif de fermeture d'appendice atrial gauche (100) selon la revendication 12, dans lequel le composant de détection (18) comprend un système hydraulique comportant une valve à faible débit, dans lequel, en réponse à une variation positive de la pression atriale gauche, le composant de détection (18) déplace le fluide à travers la valve à faible débit sous une résistance hydraulique dans une première direction soumise à une constante de temps du composant de détection définie au moins en partie par la valve à faible débit.

15. Dispositif de fermeture d'appendice atrial gauche (100) selon la revendication 12, dans lequel, en réponse à une variation négative de la pression atriale gauche, le composant de détection (18) déplace le fluide à travers la valve à faible débit dans une seconde direction opposée à la première direction en fonction de la constante de temps du composant de détection (18).
